# EUROPEAN PATENT SPECIFICATION

(11) **EP 2 558 079 B1**
(45) Date of publication and mention of the grant of the patent: **22.05.2019**
(21) Application number: 10778730.1
(22) Date of filing: 03.06.2010
(51) Int. Cl.: A61K 9/50, A61K 9/00, A61K 31/4439

(54) **CIPROFLOXACIN DRY SYRUP COMPOSITION**
TROCKENSIRUPZUSAMMENSETZUNG AUS CIPROFLOXACIN
COMPOSITION SÈCHE DE CIPROFLOXACINE POUR SUSPENSION BUVABLE

(30) Priority: 12.04.2010 IN 1199MU2010
(43) Date of publication of application: 20.02.2013
(73) Proprietor: S. Zhaveri Pharmakem PVT. Ltd, Mumbai 400 018 Maharashtra (IN)
(72) Inventor: JAVERI, Uma, Rajan, Mumbai 400 018 Maharashtra (IN)
(74) Representative: Rees, Kerry
(86) International application number: PCT/IN2010/000367
(87) International publication number: WO 2011/128906

(56) References cited:
- WO-A1-01/35930
- WO-A1-2004/087111
- US-A- 5 695 784
- US-A1- 2002 197 327
- US-B1- 6 565 877
- FRIEND D R: "POLYACRYLATE RESIN MICROCAPSULES FOR TASTE MASKING OF ANTIBIOTICS", JOURNAL OF MICROENCAPSULATION, TAYLOR AND FRANCIS, BASINGSTOKE, GB, vol. 9, no. 4, 1 October 1992 (1992-10-01) , pages 469-480, XP000296316, ISSN: 0265-2048

## Description

### TECHNICAL FIELD OF THE INVENTION:

The present invention relates to a process for preparation of taste masked dry syrup composition of ciprofloxacin comprising ciprofloxacin granules along with at least one pharmaceutically acceptable excipient. The present invention also relates to pharmaceutical composition prepared by the said process.

### BACKGROUND OF THE INVENTION:

Ciprofloxacin was first disclosed in US Patent No. 4670444 granted to Bayer A.G. in 1983 and subsequently approved by the United States Food and Drug Administration (FDA) in 1987. Ciprofloxacin is a drug used to treat bacterial infections. It is a second generation fluoroquinolone antibacterial. It kills bacteria by interfering with the enzymes that cause DNA to unwind and duplicate. Chemically Ciprofloxacin is known as 1-cyclopropyl- 6-fluoro- 4-oxo- 7-piperazin- 1-yl- quinoline- 3-carboxylic acid, having a following structure:

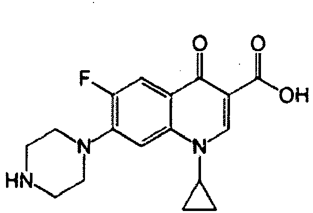

Ciprofloxacin has been used in various formulations such as tablets, granules, injection, suspension etc. but it is desirable to formulate in syrups or dry syrups for administration to children. The syrup or dry syrup preparation is suitable not only for children but also aged persons in view of easier administration. Particularly, dry syrup preparation is advantageous because it is easily weighed and packaged and further it is convenient for carrying.

US Patent No. 5695784 discloses a pharmaceutical composition containing an active ingredient which is flavour-masked by microcapsulation having a rapid release of an active ingredient and high bioavailability characterized in that the active ingredient is Ciprofloxicin and is present as the anhydrate of its base form and contains less than 5% of water in the form of water of crystallization or other water adducts and is present in an oily juice formulation and the capsule walls consist of a coating which comprises a cationic copolymer of dimethylaminoethyl methacrylate and neutral methyacrylic acid esters, neutral methyl ester and/or ethyl ester compounds of polymethacrylic acid; quaternary ammonium compounds of polymethacrylic acid, or ethylcellulose and triethylcitrate and optionally hydroxypropylmethylcellulose and the microcapsules have a size of 10 to 800 .mu.m.

US Patent No. 6136347 discloses a pharmaceutical composition comprising microcapsules, said microcapsules comprising an active ingredient microencapsulated within a microcapsule wall, wherein said active ingredient is present in said microcapsules as an anhydrate of its base form, said microcapsule wall comprises a coating of water-insoluble neutral methyl or ethyl ester compounds of polymethacrylic acid or a mixture thereof, or quaternary ammonium compounds of polymethacrylic acid or a mixture thereof, or ethylcellulose, and wherein the microcapsule wall further comprises triethylcitrate and optionally hydroxypropylmethylcellulose, and said microcapsules are free of disintegrants.

US Patent No. 6565877 discloses a taste masked composition comprising a bitter tasting drug selected from the group consisting of erythromycin, clarithromycin, ciprofloxacin, norfloxacin, cefuroxime, ceftriaxone, chlorampheniol, chloropromazine and their pharmaceutically acceptable salts and esters, and a combination of two enteric polymers comprising, a methacrylic acid copolymer and a phthalate polymer, wherein the ratio of methacrylic acid copolymer to phthalate polymer is between 1:9 or 9:1.

US Patent Publication No. 2006039981 discloses a taste-masked pharmaceutical dosage form comprising one or more drugs and one or more cationic polymers synthesized from dimethylaminoethyl methacrylate and neutral methacrylic acid esters, wherein the wt/wt ratio of the drug to polymer is less than about one to two.

US Patent Publication No. 20030133982 discloses a solid dosage form, comprising a matrix core comprising intragranular ethylcellulose and a water soluble active agent granulated and compressed together with extragranular ethylcellulose, and a film coating comprising a hydrophobic polymer, wherein the film coating completely encases the matrix core. The said solid dosage form is a tablet.

US Patent Publication No. 20020197327 discloses a taste masked pharmaceutical composition comprising a microcapsule, wherein the microcapsule comprises a pharmaceutically active agent core coated with a taste masking effective amount of a water-insoluble enteric coating, wherein the coating comprises a weakly acidic methacrylic acid-ethyl acrylate copolymer.

British Patent No. 2081092 also discloses a lipid coating for the purpose of taste masking. It was however found that wax coating resulted in poor dissolution of the active ingredients in the alimentary tract. Further the patent discloses a technique to overcome this problem by mixing the waxes with a water swellable polymer. Again the use of the water swellable polymer referred to in the patent makes it less appropriate for the liquid orals like suspensions and dry syrup.

The bitter taste of the drugs, which are orally administered, is disadvantageous in several aspects. Taste is an important parameter governing the patient compliance. The disagreeable taste of drugs causes difficulties in swallowing or causes patients to avoid their medication, thereby resulting in low patient compliance. Conventional taste masking techniques such as use of sweeteners, amino acids, flavoring agents are often unsuccessful in masking the taste of the highly bitter drugs like quinine, barberin, etoricoxib, antibiotics like levofloxacin, ofloxacin, sparfloxacin, gatifloxacin, ciprofloxacin, cefuroxime axetil, erythromycin and clarithromycin. Thus, taste-masking technologies are considered important and are developed by many researchers.

Taste masking is a major problem when the drugs are extremely unpleasant and bitter. Further, this problem is not only restricted to the liquid oral compositions like solutions, dry syrups and suspensions but may also be encountered during the formulation of chewable tablets or dispersible tablets wherein these dosage forms usually lead to perceptible exposure of active ingredient to taste buds.

Many patients, especially children and elderly, have trouble in swallowing whole tablets and even capsules. It is therefore desirable to administer drugs to such patients either as a liquid dosage form or as a fast dissolving or fast disintegrating solid dosage form. Fast dissolving or disintegrating solid dosage forms, due to their ease of administration and pleasant taste, may encourage patients to adhere to daily medication regimens and therefore provide better compliance. These dosage forms combine the advantages of both liquid and conventional tablet formulations, and also offer advantage over both traditional dosage forms. For example, they provide the convenience of a tablet formulation while also allowing the ease of swallowing provided by a liquid formulation. They also allow the luxury of much more accurate dosing than the primary alternative, oral liquids.

The processes used for taste masking in the prior arts listed above involve multiple steps which are technically complicated and difficult to reproduce, and economically disadvantageous. Therefore, there exists a need for taste masked dosage form, with higher patient compliance, economical and easy to manufacture.

Therefore, the main objective of the present invention is to prepare a taste masked dry syrup composition comprising ciprofloxacin, which is stable, having higher patient compliance, easy to manufacture and hence economical and useful for oral administration. Further the said composition is in single or multiple, easy-to-take dosage form which is mixed with water prior or suitable diluents to use.

### SUMMARY OF THE INVENTION:

In accordance with the above objective the present invention provides a process for preparing the stable taste masked dry syrup composition of ciprofloxacin as claimed in any of the preceding claims comprising the steps of:
(a) granulating the ciprofloxacin base with granulating agents and solvent;
(b) coating the granules of step (a) with the coating solution of Eudragit® E PO, a cationic copolymer based on dimethylaminoethyl methacrylate and neutral methacrylic esters having characteristic white powder with and amine-like odour, in solvent to obtain taste masked ciprofloxacin granules;
(c) mixing taste masked ciprofloxacin granules of step (b) with suspending agent, sweeteners, flavour, glidant, lubricant and buffering agent to obtain dry syrup and
(d) packing the dry syrup of step (c) into single dose sachet or multiple dose container.

In another aspect, the present invention provides a stable taste masked dry syrup composition of ciprofloxacin comprising ciprofloxacin base granules having particle size ranging from 150 to 600 µm (microns) wherein said granules are granulated with granulating agents selected from Eudragit® E PO, a cationic copolymer based on dimethylaminoethyl methacrylate and neutral methacrylic esters having characteristic white powder with and amine-like odour, HPMC, HPC and combinations and taste masking coating with Eudragit® E PO, a cationic copolymer based on dimethylaminoethyl methacrylate and neutral methacrylic esters having characteristic white powder with and amine-like odour, along with at least one pharmaceutically acceptable excipient.

A dry syrup composition of the present invention may have higher patient compliance, be easy to manufacture and hence, be economical. Further the said composition may be in single or multiple, easy-to-take dosage form.

### DETAILED DESCRIPTION OF THE INVENTION:

The invention will now be described in details in connection with certain preferred and optional embodiments so that various aspects thereof may be more fully understood and appreciated.

The present invention describes a process for preparing the stable taste masked dry syrup composition comprising ciprofloxacin in its base form for oral administration.

The present invention also describes dry syrup composition of ciprofloxacin comprising taste masked coated ciprofloxacin base granules along with at least one pharmaceutically acceptable excipients. Ciprofloxacin in its base form is present in an amount of 50 to 250mg/ml.

The present invention provides the dry syrup composition which is stable, having higher patient compliance, easy to manufacture and hence economical. Further the said composition is made available in single or multiple, easy-to-take dosage form.

In an embodiment the said single dosage form is to be mixed with 100 ml to 150 ml water prior to dispensing or consuming whereas said multiple dosage form is to be diluted with diluting fluid comprising of at least one diluting fluid selected from sorbitol solution, glycerine, sucrose syrup, xylitol, maltitol propylene glycol, polyethylene glycol and combinations thereof is mixed with flavors which is supplied along with the taste masked granules of the present invention. The diluting fluid may be present in the range of 10 to 90% of the total weight.

In the embodiments of the present invention the dry syrup composition is granulated using one or more granulating agents along with solvent. The granules so formed are coated with coating agent and solvent.

Disclosed is a stable taste masked dry syrup composition of ciprofloxacin comprising ciprofloxacin granules along with at least one pharmaceutically acceptable excipient prepared by a process comprising steps of:
(a) granulating the ciprofloxacin base with granulating agents;
(b) coating the granules of step (a) with the coating solution of methacrylate copolymer in solvent to obtain taste masked ciprofloxacin granules;
(c) mixing taste masked ciprofloxacin granules of step (b) with suspending agent, sweeteners, flavour, glidant, lubricant and buffering agent to obtain dry syrup and
(d) packing the dry syrup of step (c) into single dose sachet or multiple dose container.

The said ciprofloxacin taste masked dry syrup composition may be prepared by the process comprising of following steps:

### I) Preparation of Ciprofloxacin Granules:

a. Dissolving granulating agent in solvent, optionally mixing with diluents;
b. granulating 250 mg of Ciprofloxacin base with step (a) solution & air drying the granules at required temperature;
c. passing the dried granules through mesh size from #30 to #100;
d. preparing the coating solution by dissolving methacrylate copolymers in solvent; and
e. loading the granules of step (c) in the FBC and coating the same with solution of step (d) to obtain Ciprofloxacin taste masked granules.

### II) Preparation of Ciprofloxacin dry syrup:

a. mixing and sifting suspending agent, sweeteners, flavour, glidant, and lubricant through mesh size ranging between #30 to 100;
b. sifting buffering agent through mesh size #100 and mixing with step (a)
c. mixing ciprofloxacin taste masked granules of Part I with step (b);
d. sifting lubricant through mesh size ranging between #30 to 100 and mixing the same with step (c); and
e. packing into single dose sachet or multiple dose container.

Suitable granulating agents can be selected from Methacrylate copolymers, Hydroxy Propyl Methyl Cellulose (HPMC), Hydroxy Propyl Cellulose (HPC) and combinations thereof. The granulating agents are present in the range of 5-50% of quantity of Ciprofloxacin base.

Suitable Methacrylate copolymers may be selected from Eudragit EPO which is a cationic copolymer based on dimethylaminoethyl methacrylate and neutral methacrylic esters having characteristic white powder with an amine-like odour. In the present invention, Eudragit EPO is used in the range of 5-50 % of quantity of Ciprofloxacin base. HPMC and HPC of various grades, preferably 5cps to 15cps grade can be used as a granulating agent and in the range of 0-40% of quantity of Ciprofloxacin base.

Suitable solvent used is selected from Isopropyl alcohol (IPA), Alcohol, Methylene chloride, Water and combinations thereof. The ratio of granulating agents and solvent in the present invention is in the ratio of 1:1 to 1:7.

Optionally, other excipients such as microcrystalline cellulose, starch, Prosolve, Lactose etc are used while preparing the granules. The concentration of said excipients is in the range 0% to 15 % of the quantity of Ciprofloxacin base.

Suitable suspending agent is selected from the group of Xanthan gum, hypromellose, sodium CMC, hydroxy propyl cellulose, veegum, guar gum is in an amount of 0.1 to 0.5%.

Suitable sweeteners are selected from the group of Neotame, Sucrose, Xylitol, Aspartame, acesulfame potassium and mixtures thereof; present in an amount of 3 to 10%.

Suitable flavor is selected from mix fruit powder flavor, orange, banana, pineapple, strawberry and the like and is present in an amount of 0.2 to 1%.

Suitable buffer used is Sodium citrate, citric acid, sodium phosphate, potassium phosphate in an amount of 0.2 to 1%.

Suitable glidant is selected from the group of Aerosil, talc, titanium dioxide in an amount of 0 to 0.1%.

Suitable lubricant is selected from the group of Magnesium stearate, calcium stearate and sodium stearyl fumarate in an amount of 0.05 to 0.2%. The said lubricant helps in filling the granules in a single dose or multiple doses.

The taste masked ciprofloxacin dry syrup composition of the present invention comprises of taste masked Ciprofloxacin base granules have particle size ranging from # 30 mesh to # 100 mesh; preferably from # 40 mesh to # 80 mesh i.e. 150 to 600 microns.

The instant invention is more specifically explained by following examples. However, it should be understood that the scope of the present invention is not limited by the examples in any manner. It will be appreciated by any person skilled in this art that the present invention includes following examples and further can be modified and altered within the technical scope of the present invention.

### EXAMPLES:

### Example 1: FORMULA FOR SINGLE DOSE SACHET

**I) Ciprofloxacin Granules:**

| **Sr. No.** | **Ingredients** | **mg/ml** | **Specification** | **FUNCTION** |
|---|---|---|---|---|
| | **Part A Granulation** | | | |
| 1 | Ciprofloxacin (Base) USP | 50 | USP | API |
| 2 | Microcrystalline Cellulose(Vivapur) | 5.0 | NF | Diluent |
| 3 | HPMC 15 cps | 10.0 | USP | Binder |
| 4 | Eudragit EPO | 2.5 | USP | Binder and Taste masking agent |
| 5* | Isopropyl alcohol | Qs | USP | Solvent |
| 6 | Water | Qs | | |
| | Total | **67.5** | | |

| | **Part B Coating** | | | |
|---|---|---|---|---|
| 7 | Eudragit EPO | 38.0 | USP | Taste masking agent |
| | Talc | 3.8 | | |
| 8* | Isopropyl alcohol | qs | USP | Solvent |
| | | 109.3 | | |

| | | | | |
|---|---|---|---|---|
| *Will not appear in final formulation | | | | |

### Procedure:

1. Mixing Ciprofloxacin, MCC101(Vivapur), HPMC and Eudragit EPO in RMG;
2. granulating the mixture of step 1 using IPA: Water (1:1) for obtaining granules;
3. drying the granules of step 3 at 50°C;
4. loss on drying (NMT-2.0%)
5. passing the dried granules through #40;
6. retaining granules of step 5 on #100;

### Coating:

7. preparing coating solution by dissolving Eudragit EPO in IPA and
8. loading the granules in FBC and coating with solution of step 7 to obtain weight gain upto 70 to 72%.

The coating parameters are as follows.

| | | |
|---|---|---|
| 1 | Atomization Air | 1.5 bar |
| 2 | Blower Speed | 35 rpm |
| 3 | Inlet temperature | 40°C |
| 4 | Bed temperature | 35°C |
| 5 | Exhaust temperature | 30°C |
| 6 | Peristaltic pump speed | 5 rpm |

**II) Ciprofloxacin Dry Syrup Preparation:**

| Sr. No. | Ingredients | mg/ ml | Specification | FUNCTION |
|---|---|---|---|---|
| 1 | Ciprofloxacin TM granules | 116 | - | Taste masked granules |
| 2 | Xanthan gum | 0.2 | USP | Suspending Agent |
| 3 | Neotame | 1.0 | IH | Sweetener |
| 4 | Sucrose | 10.0 | USP | Sweetener |
| 5 | Aspartame | 1.0 | USP | Sweetener |
| 6 | Mix fruit powder flavor | 1.0 | IH | Flavour |
| 7 | Na-citrate (100 mesh) | 1.0 | USP | Buffer |
| 8 | Aerosil | 0.1 | USP | Glidant |
| 9 | Magnesium Stearate | 0.2 | USP | Lubricant |

### Procedure:

1. Sifting ingredient no. 2, 3, 4,5,6,8 and 9 through #40;
2. sifting ingredient no 7 through #100 and mixing the same with step 1;
3. mixing Ciprofloxacin TM Granules of Part-I with step 2;
4. sifting Magnesium Stearate through #60 and mixing with step 3 and
5. packing single dose sachet (600-700 mg dry syrup equivalent to 250mg ciprofloxacin)

### Example 2: FORMULA FOR MULTIPLE DOSES

**I) Ciprofloxacin Granules**

| Sr. No. | Ingredients | mg/ ml | g/100ml | Specific ation | FUNCTION |
|---|---|---|---|---|---|
| | **Part A Granulation** | | | | |
| 1 | Ciprofloxacin (Base) USP | 50 | 5.0 | USP | API |
| 2 | Microcrystalline Cellulose (Vivapur) | 5.0 | 0.5 | NF | Diluent |
| 3 | HPMC 15 cps | 10.0 | 1.0 | USP | Binder |
| 4 | Eudragit EPO | 2.5 | 0.25 | USP | Binder and Taste masking agent |
| 5* | Isopropyl alcohol | Qs | Qs | USP | Solvent |
| 6 | Water | Qs | Qs | | |
| 7 | Total | **67.5** | 6.75 | | |

| | **Part B Coating** | | | | |
|---|---|---|---|---|---|
| 8 | Eudragit EPO | 40.5 | 4.05 | USP | Taste masking agent |
| 9 | Talc | 8.1 | 0.81 | | |
| 10* | Isopropyl alcohol | qs | qs | USP | Solvent |
| | | 116 | 11.6 | | |

| | | | | | |
|---|---|---|---|---|---|
| *Will not appear in final formulation | | | | | |

### Procedure:

1. Mixing Ciprofloxacin, Microcrystalline Cellulose 101, HPMC and Eudragit EPO in RMG;
2. granulating the mixture of step 1 using IPA: Water (1:1);
3. drying the granules at 50°C;
4. loss on drying (NMT-2.0%);
5. passing the dried granules through #40;
6. retaining step 5 granules on #100;

### Coating:

7. preparing coating solution by dissolving Eudragit EPO in IPA and
8. loading the granules in the FBC and coating the above solution to obtained weight gain upto 38.24%.

The coating parameters are as follows.

| | | |
|---|---|---|
| 1 | Atomization Air | 1.5 bar |
| 2 | Blower Speed | 35 rpm |
| 3 | Inlet temperature | 40°C |
| 4 | Bed temperature | 35°C |
| 5 | Exhaust temperature | 30°C |
| 6 | Peristaltic pump speed | 5 rpm |

**II) Ciprofloxacin Dry Syrup Preparation:**

| Sr. No. | Ingredients | mg/ ml | g/100ml | Specification | FUNCTION |
|---|---|---|---|---|---|
| 1 | Ciprofloxacin TM granules | 116 | 11.6 | - | Taste masked granules |
| 2 | Xanthan gum | 0.2 | 0.02 | USP | Suspending Agent |
| 3 | Neotame | 1.0 | 0.1 | IH | Sweetener |
| 4 | Sucrose | 10. | 1.0 | USP | Sweetener |
| 5 | Aspartame | 1.0 | 0.4 | USP | Sweetener |
| 6 | Mix fruit powder flavor | 1.0 | 0.1 | IH | Flavour |
| 7 | Na-citrate (100 mesh) | 1.0 | 0.1 | USP | Buffer |
| 8 | Aerosil | 0.1 | 0.01 | USP | Glidant |
| 9 | Magnesium Stearate | 0.2 | 0.02 | USP | Lubricant |
| | Total | 130.60 | 13.06 | | |

### Procedure:

1. Sifting ingredient no. 2, 3, 4,5,6,8 and 9 through #40;
2. sifting ingredient no. 7 through #100 and mixing with step 1;
3. mixing Ciprofloxacin TM Granules of Part I with step 2.
4. sifting Magnesium Stearate through #60 and mixing the same with step 3.
5. packing multiple dose in glass bottle (130.6 g dry syrup equivalent to 5.0 g ciprofloxacin)

**III) Diluents for ciprofloxacin suspension:**

| Sr. No. | Ingredients | mg/ ml | g/100ml | Specification | FUNCTION |
|---|---|---|---|---|---|
| 1 | Glycerin | 98.9 | 9.89 | USP | Diluent |
| 2 | Sorbitol solution | 998.7 | 99.87 | USP | Diluent |
| 3 | Art Strawberry FLV F915527, FD01097 | 2.5 | 0.25 | IH | Flavour |

### Procedure:

Mixing ingredient no. 1, 2, and 3 under stirring to prepare a suspension and dispersing ciprofloxacin dry syrup in said suspension of diluents and shake well.

### Example 4: STABILITY DATA:

| |
|---|
| PRODUCT NAME: Ciprofloxacin for Oral Suspension |
| Strength: 250mg/5mL |
| Pack: Taste masked granules equivalent to 20 doses in amber colored glass bottle. Diluting fluid to reconstitute to 100 ml in HDPE bottle. |
| Storage Condition: 40°C/75%RH |

| **TEST** | **Specifications** | **Initial** | **1 months** | **2 Months** | **3 Months** |
|---|---|---|---|---|---|
| **Description** | White to Off-white granules suspended in diluting fluid | complies | complies | complies | complies |
| **pH** | Between 6.0 and 7.0 | 6.5 | 6.6 | 6.6 | 6.6 |
| **Dissolution** % Ciprofloxacin | NLT 80%(Q) after 45 minutes in 0.05M Acetate buffer+0.25% SLS, pH4.5 | 86.4 | 87.5 | 88.0 | 87.8 |
| **Assay** % ciprofloxacin | 90 % to 110% of Labeled amount | 99.8 | 100.0 | 99.5 | 99.4 |

## Claims

1. A stable taste masked dry syrup composition of ciprofloxacin comprising ciprofloxacin base granules having particle size ranging from 150 to 600 µm (microns) wherein said granules are granulated with granulating agents selected from Eudragit® E PO, a cationic copolymer based on dimethylaminoethyl methacrylate and neutral methacrylic esters having characteristic white powder with an amine-like odour, HPMC, HPC and combinations and taste masking coating with Eudragit® E PO, a cationic copolymer based on dimethylaminoethyl methacrylate and neutral methacrylic esters having characteristic white powder with and amine-like odour, along with at least one pharmaceutically acceptable excipient.

2. The stable taste masked dry syrup composition of ciprofloxacin as claimed in Claim 1, wherein the ciprofloxacin is in its base form.

3. The stable taste masked dry syrup composition of ciprofloxacin as claimed in Claim 1, wherein ciprofloxacin is in its base form incorporated in an amount of 250 mg.

4. The stable taste masked dry syrup composition of ciprofloxacin as claimed in Claim 1, wherein said granulating agents are present in the range of 5-50 % of quantity of ciprofloxacin base.

5. The stable taste masked dry syrup composition of ciprofloxacin as claimed in Claim 1, wherein other excipients used to prepare the granules are selected from microcrystalline cellulose, starch, Prosolve and lactose.

6. The stable taste masked ciprofloxacin dry syrup composition as claimed in Claim 1, wherein said dry syrup is in the form of single dose sachet added to 100 ml to 150 ml of water prior to dispensing or consuming.

7. The stable taste masked ciprofloxacin dry syrup composition as claimed in Claim 1, wherein said dry syrup is in the form of multiple dose diluted with diluting fluid supplied along with the taste masked granules.

8. The stable taste masked ciprofloxacin dry syrup composition as claimed in Claim 1, wherein said diluting fluid comprises of at least one diluent selected from sorbitol solution, glycerin, sucrose syrup, xylitol, maltitol, propylene glycol, polyethylene glycol and combinations thereof mixed with flavour.

9. A process for preparing the stable taste masked dry syrup composition of ciprofloxacin as claimed in any of the preceding claims comprising the steps of:
(a) granulating the ciprofloxacin base with granulating agents and solvent;
(b) coating the granules of step (a) with the coating solution of Eudragit® E PO, a cationic copolymer based on dimethylaminoethyl methacrylate and neutral methacrylic esters having characteristic white powder with an amine-like odour, in solvent to obtain taste masked ciprofloxacin granules;
(c) mixing taste masked ciprofloxacin granules of step (b) with suspending agent, sweeteners, flavour, glidant, lubricant and buffering agent to obtain dry syrup and
(d) packing the dry syrup of step (c) into single dose sachet or multiple dose container.

10. The process as claimed in Claim 9, wherein said solvent is selected from isopropyl alcohol (IPA), alcohol, methylene chloride, water and combinations thereof.

11. The process as claimed in Claim 9, wherein said suspending agent is selected from the group of xanthan gum, hypromellose, sodium CMC, hydroxyl propyl cellulose, guar gum, veegum.

12. The process as claimed in Claim 9, wherein said sweeteners are selected from neotame, sucrose, xylitol, aspartame, acesulfame potassium and mixtures thereof.

13. The process as claimed in Claim 9, wherein said lubricant is selected from magnesium stearate, calcium stearate and sodium stearyl fumarate.

## Patentansprüche

1. Stabile geschmacksmaskierte Trockensirupzusammensetzung von Ciprofloxacin, die Folgendes umfasst: Granulat der Ciprofloxacin-Base, das eine Partikelgröße im Bereich von 150 bis 600 µm (Mikron) aufweist, wobei das Granulat mit Granulierungsmitteln granuliert wird, die aus Folgenden ausgewählt sind: Eudragit® E PO, einem kationischen Copolymer basierend auf Dimethylaminoethylmethacrylat und neutralen Methacrylestern, das ein charakteristisches weißes Pulver mit einem aminartigen Geruch ist, HPMC, HPC und Kombinationen, und geschmacksmaskierende Beschichtung mit Eudragit® E PO, einem kationischen Copolymer basierend auf Dimethylaminoethylmethacrylat und neutralen ethacrylestern, das ein charakteristisches weißes Pulver mit einem aminartigen Geruch ist, zusammen mit mindestens einem pharmazeutisch verträglichen Hilfsstoff.

2. Stabile geschmacksmaskierte Trockensirupzusammensetzung von Ciprofloxacin nach Anspruch 1, wobei das Ciprofloxacin in seiner Basenform vorliegt.

3. Stabile geschmacksmaskierte Trockensirupzusammensetzung von Ciprofloxacin nach Anspruch 1, wobei Ciprofloxacin in seiner Basenform in einer Menge von 250 mg eingefügt ist.

4. Stabile geschmacksmaskierte Trockensirupzusammensetzung von Ciprofloxacin nach Anspruch 1, wobei die Granulierungsmittel im Bereich von 5-50 % der Menge der Ciprofloxacin-Base vorliegen.

5. Stabile geschmacksmaskierte Trockensirupzusammensetzung von Ciprofloxacin nach Anspruch 1, wobei andere Hilfsstoffe, die zur Herstellung des Granulats verwendet werden, aus mikrokristalliner Cellulose, Stärke, Prosolve und Lactose ausgewählt sind.

6. Stabile geschmacksmaskierte Ciprofloxacin-Trockensirupzusammensetzung nach Anspruch 1, wobei der Trockensirup in Form eines Einzeldosis-Päckchens vorliegt, das zu 100 ml bis 150 ml Wasser vor dem Ausgeben oder Konsumieren zugegeben wird.

7. Stabile geschmacksmaskierte Ciprofloxacin-Trockensirupzusammensetzung nach Anspruch 1, wobei der Trockensirup in Form einer Mehrfachdosis vorliegt, die mit Verdünnungsflüssigkeit verdünnt wird, die zusammen mit dem geschmacksmaskierten Granulat bereitgestellt ist.

8. Stabile geschmacksmaskierte Ciprofloxacin-Trockensirupzusammensetzung nach Anspruch 1, wobei die Verdünnungsflüssigkeit mindestens ein Verdünnungsmittel umfasst, das aus Folgenden ausgewählt ist: Sorbitollösung, Glycerin, Saccharosesirup, Xylitol, Maltit, Propylenglycol, Polyethylenglycol und Kombinationen davon, die mit Aroma gemischt sind.

9. Verfahren für die Herstellung der stabilen geschmacksmaskierten Trockensirupzusammensetzung von Ciprofloxacin nach einem der vorstehenden Ansprüche, das die folgenden Schritte umfasst:
(a) Granulieren der Ciprofloxacin-Base mit Granulierungsmitteln und Lösungsmittel;
(b) Beschichten des Granulats von Schritt (a) mit der Beschichtungslösung von Eudragit® E PO, einem kationischen Copolymer basierend auf Dimethylaminoethylmethacrylat und neutralen Methacrylestern, das ein charakteristisches weißes Pulver mit einem aminartigen Geruch ist, in Lösungsmittel, um geschmacksmaskiertes Ciprofloxacin-Granulat zu erhalten;
(c) Mischen des geschmacksmaskierten Ciprofloxacin-Granulats von Schritt (b) mit Suspensionsmittel, Süßungsmitteln, Aroma, Gleitmittel, Schmiermittel und Puffermittel, um Trockensirup zu erhalten, und
(d) Verpacken des Trockensirups von Schritt (c) in Einzeldosis-Päckchen oder Mehrfachdosis-Behälter.

10. Verfahren nach Anspruch 9, wobei das Lösungsmittel aus Isopropylalkohol (IPA), Alkohol, Methylenchlorid, Wasser und Kombinationen davon ausgewählt ist.

11. Verfahren nach Anspruch 9, wobei das Suspensionsmittel aus der Gruppe von Xanthan-Gummi, Hypromellose, Natrium-CMC, Hydroxylpropylcellulose, Guar-Gummi, Veegum ausgewählt ist.

12. Verfahren nach Ansprüche 9, wobei die Süßungsmittel aus Neotam, Saccharose, Xylitol, Aspartam, Acesulfam-Kalium und Mischungen davon ausgewählt sind.

13. Verfahren nach Ansprüche 9, wobei das Schmiermittel aus Magnesiumstearat, Calciumstearat und Natriumstearylfumarat ausgewählt ist.

## Revendications

1. Composition de sirop sec à goût masqué stable, à base de ciprofloxacine, comprenant des granulés à base de ciprofloxacine ayant une taille de particule allant de 150 à 600 µm (microns), où lesdits granulés sont granulés avec des agents de granulation choisis parmi Eudragit® E PO, un copolymère cationique à base de méthacrylate de diméthylaminoéthyle et d'esters méthacryliques neutres se présentant comme une poudre blanche caractéristique ayant une odeur de type amine, la HPMC, la HPC et des combinaisons et un revêtement de masquage de goût par Eudragit® E PO, un copolymère cationique à base de méthacrylate de diméthylaminoéthyle et d'esters méthacryliques neutres se présentant comme une poudre blanche caractéristique ayant une odeur de type amine, conjointement avec au moins un excipient pharmaceutiquement acceptable.

2. Composition de sirop sec à goût masqué stable, à base de ciprofloxacine, selon la revendication 1, dans laquelle la ciprofloxacine se trouve sous sa forme de base.

3. Composition de sirop sec à goût masqué stable, à base de ciprofloxacine, selon la revendication 1, dans laquelle la ciprofloxacine se trouve sous sa forme de base, incorporée selon une quantité de 250 mg.

4. Composition de sirop sec à goût masqué stable, à base de ciprofloxacine, selon la revendication 1, dans laquelle lesdits agents de granulation sont présents dans une plage de 5-50% de quantité de base de ciprofloxacine.

5. Composition de sirop sec à goût masqué stable, à base de ciprofloxacine, selon la revendication 1, dans laquelle d'autres excipients utilisés afin de préparer les granulés sont choisis parmi la cellulose microcristalline, l'amidon, le Prosolve et le lactose.

6. Composition de sirop sec, à base de ciprofloxacine, à goût masqué stable selon la revendication 1, dans laquelle ledit sirop sec se trouve sous la forme d'un sachet de dose unique ajouté à de 100 ml à 150 ml d'eau préalablement à la distribution ou la consommation.

7. Composition de sirop sec, à base de ciprofloxacine, à goût masqué stable selon la revendication 1, dans laquelle ledit sirop sec se trouve sous la forme de doses multiples diluées par un fluide de dilution fourni conjointement avec les granulés à goût masqué.

8. Composition de sirop sec, à base de ciprofloxacine, à goût masqué stable selon la revendication 1, dans laquelle ledit fluide de dilution comprend au moins un diluant choisi parmi une solution de sorbitol, le glycérol, un sirop de saccharose, le xylitol, le maltitol, le propylène glycol, le polyéthylène glycol et des combinaisons de ceux-ci mélangés avec un arôme.

9. Procédé de préparation de la composition de sirop sec à goût masqué stable, à base de ciprofloxacine, selon l'une quelconque des revendications précédentes, comprenant les étapes
(a) de granulation de la base de ciprofloxacine avec des agents de granulation et un solvant ;
(b) de revêtement des granulés de l'étape (a) par la solution de revêtement d'Eudragit® E PO, un copolymère cationique à base de méthacrylate de diméthylaminoéthyle et d'esters méthacryliques neutres se présentant comme une poudre blanche caractéristique ayant une odeur de type amine, dans un solvant, afin d'obtenir des granulés de ciprofloxacine à goût masqué ;
(c) de mélange des granulés de ciprofloxacine à goût masqué de l'étape (b) avec un agent de mise en suspension, des édulcorants, un arôme, un agent glissant, un lubrifiant et un agent tampon, afin d'obtenir un sirop sec ; et
(d) d'emballage du sirop sec de l'étape (c) dans un sachet de dose unique ou un conteneur à doses multiples.

10. Procédé selon la revendication 9, dans lequel ledit solvant est choisi parmi l'alcool isopropylique (IPA), un alcool, le chlorure de méthylène, l'eau et des combinaisons de ceux-ci.

11. Procédé selon la revendication 9, dans lequel ledit agent de mise en suspension est choisi dans le groupe constitué par la gomme xanthane, l'hypromellose, la CMC de sodium, l'hydroxypropylcellulose, la gomme guar, le Veegum.

12. Procédé selon la revendication 9, dans lequel lesdits édulcorants sont choisis parmi le néotame, le saccharose, le xylitol, l'aspartame, l'acésulfame de potassium et des mélanges de ceux-ci.

13. Procédé selon la revendication 9, dans lequel ledit lubrifiant est choisi parmi le stéarate de magnésium, le stéarate de calcium et le stéarylfumarate de sodium.
